(19) **Europäisches Patentamt** / **European Patent Office** / **Office européen des brevets**

(11) **EP 3 675 642 B1**

(12) **EUROPEAN PATENT SPECIFICATION**

(45) Date of publication and mention
of the grant of the patent:
**18.12.2024 Bulletin 2024/51**

(21) Application number: **18759640.8**

(22) Date of filing: **30.08.2018**

(51) International Patent Classification (IPC):
*A23C 9/123* (2006.01)    *A23C 9/127* (2006.01)
*A23C 13/16* (2006.01)    *A23C 17/02* (2006.01)
*A23C 19/032* (2006.01)

(52) Cooperative Patent Classification (CPC):
**A23C 9/1238;** A23C 9/127; A23C 13/16;
A23C 17/02; A23C 19/0323

(86) International application number:
**PCT/EP2018/073382**

(87) International publication number:
**WO 2019/043115 (07.03.2019 Gazette 2019/10)**

(54) **PROCESS FOR PRODUCING A MESOPHILIC FERMENTED MILK PRODUCT**

VERFAHREN ZUR HERSTELLUNG EINES MESOPHILISCH FERMENTIERTEN MILCHPRODUKTS

PROCÉDÉ DE FABRICATION D'UN PRODUIT À BASE DE LAIT FERMENTE MÉSOPHILE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.08.2017 EP 17188549**

(43) Date of publication of application:
**08.07.2020 Bulletin 2020/28**

(73) Proprietor: **Chr. Hansen A/S
2970 Hoersholm (DK)**

(72) Inventors:
• **BLOC, Virginie**
  **91292 Arpajon (FR)**
• **JANZEN, Thomas**
  **2970 Hoersholm (DK)**
• **BIRKELUND, Mimi**
  **2970 Hoersholm (DK)**
• **JIMENEZ, Luciana**
  **91292 Arpajon (FR)**
• **ODINOT, Jean-Marie**
  **91292 Arpajon (FR)**
• **PIROIS-BLIN, Sabrina**
  **91292 Arpajon (FR)**
• **GULDAGER, Helle Skov**
  **2970 Hoersholm (DK)**

(56) References cited:
**EP-A1- 2 957 180       WO-A1-2017/005601
US-A1- 2017 135 360**

**Description**

**FIELD OF THE INVENTION**

The present invention relates to a process for producing a fermented milk product.

**BACKGROUND OF THE INVENTION**

Mesophilic fermented milk products are produced at a temperature between about 22°C and about 35°C, and typically the mesophilic lactic acid bacteria Lactococcus spp. and Leuconostoc spp. are used. Mesophilic fermented milk products include buttermilk, sour milk, cultured milk, smetana, sour cream, Kefir and fresh cheese, such as quark, tvarog and cream cheese.

EP-A1-2 957 180 discloses a method of producing a fermented milk product using lactose-deficient lactic acid bacteria, in particular lactose-deficient *Streptococcus thermophilus* strains and *Lactobacillus delbrueckii* subsp. *bulgaricus* strains. US-A1-2017/135360 relates to methods of producing fermented milk products which provide an improved control of post acidification, thereby eliminating the need to rapidly cool the fermented milk product at a predetermined pH value to terminate fermentation.

**SUMMARY OF THE INVENTION**

The object of the present invention is to provide an improved process for producing a mesophilic fermented milk product.

The object of the present invention is obtained by a process for producing a fermented milk product comprising the steps of

1) adding a starter culture of lactic acid bacteria comprising at least one lactose-deficient *Streptococcus thermophilus* strain, which is capable of metabolizing a non-lactose carbohydrate, and at least one lactose-deficient *Lactococcus lactis* strain, which is capable of metabolizing the non-lactose carbohydrate, to a milk base, and

2) fermenting the milk for a period of time until a target pH is reached to obtain a fermented milk product,

wherein non-lactose carbohydrate is added to the milk base at the start of the fermentation step.

Lactose-deficient lactic acid bacteria typically grow on a non-lactose carbohydrate source, such as sucrose, galactose and glucose, added to the milk in an amount measured so as stop the fermentation process and the growth of the lactic acid bacteria by depletion of the added carbohydrate source. Hereby, the post-acidification during subsequent storage is lowered significantly or even fully prevented.

The present invention is based on the recognition that it is possible to reduce or avoid post-acidification during storage in a mesophilic fermented milk product by using a combination of a mesophilic lactose-deficient *Lactococcus lactis* strain, and a lactose-deficient *Streptococcus thermophilus* strain.

The present invention is further based on the experimental finding that the use of the said combination of strains also has a number of advantages in the production of the mesophilic fermented milk product. Firstly, in the process for producing a mesophilic fermented milk product the use of the said combination of strains allows omitting the step of cooling the fermented milk product after fermentation and before filling into end consumer cups. Thus, since no or only little post-acidification takes place after the termination of the fermentation due to the depletion of the carbohydrate growth source, cooling in order to reduce post-acidification is not necessary. Secondly, it has surprisingly been found that the said combination of strains produces an improved texture of the mesophilic fermented milk product as compared to a corresponding culture blend comprising lactose-positive strains.

**FIGURES**

Fig. 1 shows the acidification profiles of cultures C1-C4 at 34 °C.
Fig. 2 shows the acidification profiles of cultures C1-C4 at 30 °C.
Fig. 3 shows the acidification profile of culture C5 at 34 °C.
Fig. 4 shows the acidification profile of culture C6 at 34 °C.
Fig. 5 shows the acidification profiles of culture blends LC5 + ST1 and LC7 + ST1 at 30°C.
Fig. 6 shows the acidification profiles of culture blends LACcr1 + ST1 and LACcr2 + ST1 at 30°C.
Fig. 7 shows the acidification profiles of culture blends C1- C4 and the reference at 30°C.

Fig. 8 shows the acidification profiles of culture blends C1- C4 and the reference at 35°C.
Fig 9 shows the acidification profiles of C1- C3 and the reference at 30°C.
Fig 10 shows the acidification profiles of C1- C3 and the reference at 35°C

## DETAILED DISCLOSURE OF THE INVENTION

### Lactose-deficient lactic acid bacteria

The terms "deficiency in lactose metabolism" and "lactose deficient" are used in the context of the present invention to characterize LAB which either partially or completely lost the ability to use lactose as a source for cell growth or maintaining cell viability. Respective LAB are capable of metabolizing one or several carbohydrates selected from sucrose, galactose and/or glucose or another fermentable carbohydrate. Since these carbohydrates are not naturally present in milk in sufficient amounts to support fermentation by lactose deficient mutants, it is necessary to add these carbohydrates to the milk. Lactose deficient and partially deficient LAB can be characterized as white colonies on a medium containing lactose and X-Gal.

In a particular embodiment of the invention, the lactose-deficient strain is capable of metabolizing a non-lactose carbohydrate selected from the group consisting of sucrose, galactose and glucose, preferably sucrose. In a particular embodiment of the invention, the lactose-deficient strain is capable of metabolizing galactose.

In a particular embodiment of the invention, the lactose-deficient *Lactococcus lactis* subsp. *lactis* strain is sucrose-positive.

In a particular embodiment of the invention, the lactose-deficient *Lactococcus lactis* subsp. *lactis* strain is glucose-positive.

In a particular embodiment of the invention, the lactose-deficient *Streptococcus thermophilus* strain is selected from the group consisting of:

(a) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952;
(b) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953;
(c) the strain deposited with DSMZ-Deutsche Sammlung von Mikroorganismen und Zellkulturen GmbH, Inhoffenstr. 7B, D-38124 Braunschweig, on 2017-08-22 under the accession no. DSM 32599; and
(d) the strain deposited with DSMZ-Deutsche Sammlung von Mikroorganismen und Zellkulturen GmbH, Inhoffenstr. 7B, D-38124 Braunschweig, on 2017-08-22 under the accession no. DSM 32600.

In a particular embodiment of the invention, the lactose-deficient *Streptococcus thermophilus* strain is selected from the group consisting of:

(a) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952; and
(b) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953.

In a particular embodiment of the invention, the lactose-deficient *Streptococcus thermophilus* strain is selected from the group consisting of:

(c) the strain deposited with DSMZ-Deutsche Sammlung von Mikroorganismen und Zellkulturen GmbH, Inhoffenstr. 7B, D-38124 Braunschweig, on 2017-08-22 under the accession no. DSM 32599; and
(d) the strain deposited with DSMZ-Deutsche Sammlung von Mikroorganismen und Zellkulturen GmbH, Inhoffenstr. 7B, D-38124 Braunschweig, on 2017-08-22 under the accession no. DSM 32600; and.

In a particular embodiment of the invention, the *Lactococcus lactis* strain is selected from the group consisting of a *Lactococcus lactis* subsp. *cremoris* strain and a *Lactococcus lactis* subsp. *lactis* strain.

In a particular embodiment of the invention, the lactose-deficient *Lactococcus lactis* strain is selected from the group consisting of

1) the strain deposited at DSMZ under the accession number DSM 32398,
2) the strain deposited at DSMZ under the accession number DSM 18882,
3) the strain deposited at DSMZ under the accession number DSM 32399,

4) the strain deposited at DSMZ under the accession number DSM 18893,
5) the strain deposited at DSMZ under the accession number DSM 32601,
6) the strain deposited at DSMZ under the accession number DSM 32602,
7) the strain deposited at DSMZ under the accession number DSM 32603,
8) the strain deposited at DSMZ under the accession number DSM 32604,
9) the strain deposited at DSMZ under the accession number DSM 32605,
10) the strain deposited at DSMZ under the accession number DSM 32829,
11) the strain deposited at DSMZ under the accession number DSM 32830, and
12) the strain deposited at DSMZ under the accession number DSM 32832.

In a particular embodiment of the invention, the lactose-deficient *Lactococcus lactis* strain is selected from the group consisting of

1) the strain deposited at DSMZ under the accession number DSM 32398,
2) the strain deposited at DSMZ under the accession number DSM 18882,
3) the strain deposited at DSMZ under the accession number DSM 32399,
4) the strain deposited at DSMZ under the accession number DSM 18893,
5) the strain deposited at DSMZ under the accession number DSM 32829, and
6) the strain deposited at DSMZ under the accession number DSM 32830.

The above strains 1) to 6) are glucose-positive and sucrose-negative.

In a particular embodiment of the invention, the lactose-deficient *Lactococcus lactis* subsp. *cremoris* strain is selected from the group consisting of

1) the strain deposited at DSMZ under the accession number DSM 32398,
2) the strain deposited at DSMZ under the accession number DSM 18882,
3) the strain deposited at DSMZ under the accession number DSM 18893,
4) the strain deposited at DSMZ under the accession number DSM 32829, and
5) the strain deposited at DSMZ under the accession number DSM 32830.

In a particular embodiment of the invention, the lactose-deficient *Lactococcus lactis* subsp. *lactis* strain is selected from the group consisting of

1) the strain deposited at DSMZ under the accession number DSM 32399.

In a particular embodiment of the invention, the lactose-deficient *Lactococcus lactis* subsp. *lactis* strain is selected from the group consisting of

1) the strain deposited at DSMZ under the accession number DSM 32601,
2) the strain deposited at DSMZ under the accession number DSM 32602,
3) the strain deposited at DSMZ under the accession number DSM 32603,
4) the strain deposited at DSMZ under the accession number DSM 32604,
5) the strain deposited at DSMZ under the accession number DSM 32605, and
6) the strain deposited at DSMZ under the accession number DSM 32832.

The above strains 1) to 6) are glucose-negative and sucrose-positive. The said strains 1) to 6) are preferred in that when used in the process of the invention they produce fermented milk products with increased smoothness.

## Steps of the process of the invention

In a particular embodiment of the invention the lactose-deficient strains are capable of metabolizing a non-lactose carbohydrate selected from the group consisting of sucrose, galactose and glucose, preferably sucrose.

In a particular embodiment of the invention, the non-lactose carbohydrate is added to the milk base at the start of the fermentation step.

In a particular embodiment of the invention, the fermentation step is terminated by a method selected from the group consisting of 1) acidification of the fermented milk rendering at least one strain of the starter culture unable to grow, 2) cooling treatment and 3) depletion of the non-lactose carbohydrate.

Preferably, the non-lactose carbohydrate is added to the milk base in an amount measured so as to become depleted and hence result in stopping the growth of lactic acid bacteria and in stopping the fermentation. Preferably, the non-lactose carbohydrate is added to the milk base in an amount measured so as to become depleted at the target pH and hence result in stopping the growth of lactic acid bacteria and in stopping the fermentation.

The amount of non-lactose carbohydrate to be added to the milk base depends on a number of parameters, including the lactic acid bacteria strains used in the starter culture, the composition of the milk base, the fermentation temperature and the desired target pH. The amount of non-lactose carbohydrate to be added to the milk base can be determined by experimentation, and it is well within the skills of a skilled person to carry out such experimentation.

In a particular embodiment of the invention the target pH is between 3.2 and 4.8, more preferably between 4.0 and 5.2, more preferably between 4.2 and 5.0 and most preferably between 4.4 and 4.8.

In a particular embodiment of the invention the fermentation temperature is between 15°C and 35°C, preferably between 24°C and 35°C, more preferably between 26°C and 35°C, more preferably between 28°C and 35°C, and more preferably between 30°C and 34°C.

In a particular embodiment of the invention the fermented milk product is not subjected to a cooling step after the end of the fermentation step and before packaging.

In a particular embodiment of the invention, the fermented milk product is packaged at a temperature between 15 and 45°C.

In a particular embodiment of the invention, the pH value of the fermented milk product is maintained within a range of 0.3 pH units, preferably within a range of 0.2 pH units and most preferably within a range of 0.1 pH units, when stored after termination of the fermentation at the temperature used for fermentation over a period of 20 hours.

In a particular embodiment of the invention, the amount of added non-lactose carbohydrate is from 1 mg/g to 30 mg/g, preferably from 2 mg/g to 20 mg/g, and more preferably from 3 mg/g to 10 mg/g milk base.

In a particular embodiment of the invention, the amount of added non-lactose carbohydrate is from 0.1 % to 10 %, preferably from 0.2 % to 8 %, preferably from 0.3 % to 2 %, preferably from 0.4 % to 1.5 %, and more preferably from 0.5 % to 1.2 %, wherein % is (w/w) based on milk base.

In a particular embodiment of the invention the starter culture further contains one or more strains selected from the group consisting of *Lactococcus lactis* subsp. *lactis* biovar. *diacetylactis, Leuconostoc spp.* and *Bifidobacterium spp.* Also, the starter culture may contain a yeast. In a particular embodiment of the invention, the *Leuconostoc spp.* is selected from the group consisting of *Leuconostoc mesenteroides* and *Leuconostoc pseudomesenteroides.* In a particular embodiment of the invention, the *Bifidobacterium spp.* is selected from the group consisting of *Bifidobacterium longum, Bifidobacterium adolescentis, Bifidobacterium bifidum, Bifidobacterium breve, Bifidobacterium animalis* subsp. *lactis, Bifidobacterium dentium, Bifidobacterium catenulatum, Bifidobacterium angulatum, Bifidobacterium magnum, Bifidobacterium pseudocatenulatum* and *Bifidobacterium infantis*.

In a preferred embodiment of the invention, the milk base at the start of the fermentation step has a content of lactose of between 30.0 mg/ml and 70 mg/ml, preferably between 35 mg/ml and 65 mg/ml, more preferably between 40 mg/ml and 60 mg/ml, and most preferably between 50 mg/ml and 60 mg/ml.

### Fermented milk product

The present invention further relates to a fermented milk product produced by the process of the invention.

In the invention, the fermented milk product is a product produced by the process of the invention, which is produced using a starter culture of lactic acid bacteria strain comprising at least one lactose-deficient *Streptococcus thermophilus* strain and at least one lactose-deficient *Lactococcus lactis* strain.

In a particular embodiment of the invention, the fermented milk product is selected from the group consisting of buttermilk, sour milk, cultured milk, Smetana, sour cream, thick cream, cultured cream, ymer, fermented whey, Kefir, and fresh cheese, such as Quark, tvarog and cream cheese. In particular, the fermented milk product is selected from the group consisting of Quark, sour cream and Kefir.

In a preferred embodiment of the invention, the fermented milk product contains a further food product selected from the group consisting of fruit beverage, cereal products, fermented cereal products, chemically acidified cereal products, soy milk products, fermented soy milk products and any mixture thereof.

The fermented milk product typically contains protein in a level of between 1.0 % by weight to 12.0 % by weight, preferably between 2.0 % by weight to 10.0 % by weight. In a particular embodiment, sour cream contains protein in a level of between 1.0 % by weight to 5.0 % by weight, preferably between 2.0 % by weight to 4.0 % by weight. In a particular embodiment, Quark contains protein in a level of between 4.0 % by weight to 12.0 % by weight, preferably between 5.0 % by weight to 10.0 % by weight.

### Composition of the invention

The present invention further relates to a composition of lactic acid bacteria comprising at least one lactose-deficient *Streptococcus thermophilus* strain and at least one lactose-deficient *Lactococcus lactis* strain.

In a particular embodiment, the composition contains at least one lactose-deficient *Streptococcus thermophilus* strain and one lactose-deficient *Lactococcus lactis* strain. In a particular embodiment, the composition contains one lactose-deficient *Streptococcus thermophilus* strain and at least one lactose-deficient *Lactococcus lactis* strain.

In a particular embodiment, the composition contains two or more lactose-deficient *Streptococcus thermophilus* strains and at least one lactose-deficient *Lactococcus lactis.* In a particular embodiment, the composition contains at least one lactose-deficient *Streptococcus thermophilus* strain and two or more lactose-deficient *Lactococcus lactis* strains.

In a particular embodiment, the composition contains two or more lactose-deficient *Streptococcus thermophilus* strains and one lactose-deficient *Lactococcus lactis.* In a particular embodiment, the composition contains one lactose-deficient *Streptococcus thermophilus* strain and two or more lactose-deficient *Lactococcus lactis* strains.

In a particular embodiment, the composition contains two lactose-deficient *Streptococcus thermophilus* strains and one lactose-deficient *Lactococcus lactis.* In a particular embodiment, the composition contains one lactose-deficient *Streptococcus thermophilus* strain and two lactose-deficient *Lactococcus lactis* strains.

In a particular embodiment of the invention, the composition contains the strain deposited at DSMZ under the accession number DSM 32398, and the strain deposited at DSMZ under the accession number DSM 18882.

In a particular embodiment of the invention, the composition contains the strain deposited at DSMZ under the accession number DSM 32398, and the strain deposited at DSMZ under the accession number DSM 18893.

## Use of the invention

The present invention further relates to use in a process for producing a fermented milk product comprising the steps of

1) adding a starter culture of lactic acid bacteria strain to a milk base, and
2) fermenting the milk for a period of time until a target pH is reached to obtain a fermented milk product,

of a starter culture comprising at least one lactose-deficient *Streptococcus thermophilus* strain, which is capable of metabolizing a non-lactose carbohydrate, and at least one lactose-deficient *Lactococcus lactis* strain, which is capable of metabolizing a non-lactose carbohydrate.

A particular embodiment of the use of the invention is directed to use to increase the texture measured as shear stress or gel firmness of the fermented milk product as compared to using a starter culture comprising at least one lactose-positive *Streptococcus thermophilus* strain, which is capable of metabolizing lactose, and at least one lactose-positive *Lactococcus lactis* strain, which is capable of metabolizing lactose.

## Definitions

In connection with the present invention the following definitions apply:
The expression "lactic acid bacteria" ("LAB") designates a gram-positive, microaerophilic or anaerobic bacteria, which ferment sugars with the production of acids including lactic acid as the predominantly produced acid, acetic acid and propionic acid. The industrially most useful lactic acid bacteria are found within the order "Lactobacillales" which includes *Lactococcus spp., Streptococcus spp., Lactobacillus spp., Leuconostoc spp., Pseudoleuconostoc* spp., *Pediococcus spp., Brevibacterium spp., Enterococcus spp.* and *Propionibacterium spp.* These are frequently used as food cultures alone or in combination with other lactic acid bacteria.

Lactic acid bacteria, including bacteria of the species Lactobacillus sp. and Lactococcus sp., are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product or a cheese. Such lactic acid bacterial cultures are in general referred to as "starter cultures" or "starters". Typically, a starter culture for yogurt comprises *Streptococcus thermophilus* and *Lactobacillus delbrueckii* subsp. *bulgaricus,* and in most countries a yogurt is by legislation defined as a fermented milk product produced using a starter culture comprising the two said strains.

The term "milk" is to be understood as the lacteal secretion obtained by milking of any mammal, such as cows, sheep, goats, buffaloes or camels. In a preferred embodiment, the milk is cow's milk. The term milk also includes protein/fat solutions made of plant materials, e.g. soy milk.

The term "milk base" may be any raw and/or processed milk material that can be subjected to fermentation according to the method of the invention. Thus, useful milk bases include, but are not limited to, solutions/-suspensions of any milk or milk like products comprising protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk base may originate from any mammal, e.g. being substantially pure mammalian milk, or reconstituted milk powder.

Prior to fermentation, the milk base may be homogenized and pasteurized according to methods known in the art.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk base to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

"Fermentation" in the methods of the present invention means the conversion of carbohydrates into alcohols or acids through the action of a microorganism. Preferably, fermentation in the methods of the invention comprises conversion of lactose to lactic acid.

Fermentation processes to be used in production of dairy products are well known and the person of skill in the art will know how to select suitable process conditions, such as temperature, oxygen, amount and characteristics of microorganism(s) and process time. Obviously, fermentation conditions are selected so as to support the achievement of the present invention, i.e. to obtain a dairy product in solid (such as a cheese) or liquid form (such as a fermented milk product).

The expression "fermented milk product" means a food or feed product wherein the preparation of the food or feed product involves fermentation of a milk base with a lactic acid bacterium. "Fermented milk product" as used herein includes but is not limited to products such as thermophilic fermented milk products, e.g. yoghurt, mesophilic fermented milk products, e.g. sour cream and buttermilk, as well as fermented whey.

The term "thermophile" herein refers to microorganisms that thrive best at temperatures above 35°C. The industrially most useful thermophilic bacteria include Streptococcus spp. and Lactobacillus spp. The term "thermophilic fermentation" herein refers to fermentation at a temperature above about 35°C, such as between about 35°C to about 45°C. The term "thermophilic fermented milk product" refers to fermented milk products prepared by thermophilic fermentation of a thermophilic starter culture and include such fermented milk products as set-yoghurt, stirred-yoghurt and drinking yoghurt, e.g. Yakult.

The term "mesophile" herein refers to microorganisms that thrive best at moderate temperatures (15°C-35°C). The industrially most useful mesophilic bacteria include Lactococcus spp. and Leuconostoc spp. The term "mesophilic fermentation" herein refers to fermentation at a temperature between about 22°C and about 35°C. The term "mesophilic fermented milk product" refers to fermented milk products prepared by mesophilic fermentation of a mesophilic starter culture and include such fermented milk products as buttermilk, sour milk, cultured milk, Smetana, sour cream, thick cream, cultured cream, ymer, fermented whey, Kefir, Yakult and fresh cheese, such as Quark, tvarog and cream cheese.

In connection with the present invention, "shear stress" may be measured by the following method:

Seven days after production, the fermented milk product was brought to 13°C and manually stirred gently by means of a spoon (5 times) until homogeneity of the sample. The rheological properties of the sample were assessed on a rheometer (Anton Paar Physica Rheometer with ASC, Automatic Sample Changer, Anton Paar® GmbH, Austria) by using a bob-cup. The rheometer was set to a constant temperature of 13 °C during the time of measurement. Settings were as follows:

Holding time (to rebuild to somewhat original structure)

5 minutes without any physical stress (oscillation or rotation) applied to the sample. Oscillation step (to measure the elastic and viscous modulus, G' and G", respectively, therefore calculating the complex modulus G*)

$$\text{Constant strain} = 0.3\,\%,\ \text{frequency}\ (f) = [0.5...8]\ \text{Hz}$$

6 measuring points over 60 s (one every 10 s)

Rotation step (to measure shear stress at 300 1/s)

Two steps were designed:
Shear rate = [0.3-300] 1/s and 2) Shear rate = [275-0.3] 1/s.

Each step contained 21 measuring points over 210 s (on every 10 s).

The shear stress at 300 1/s was chosen for further analysis, as this correlates to mouth thickness when swallowing a fermented milk product.

In connection with the present invention, "gel firmness" may be measured by the following method:

Gel firmness is measured by a back extrusion test with a texture analyzer (TA.XT Plus, Stable Micro System, Surrey, UK) supplied with a 35mm parallel plate. The travel distance is set to 15 mm, and the travel speed to 2 mm/s. The test is performed after 7 days from production. The fermented milk product was brought to 13°C and manually stirred gently and measured in a 250 g plastic container. The maximal force (N or g) obtained by force versus distance curves is used as "gel firmness" parameter, the positive area (N*mm) as degree of deformation, the maximal negative force (N) as ropiness.

The term "low pH stable lactase" herein refers to a lactase, which retains its activity at a pH of 5.0 and a temperature of 37 $^0$C at a level of at least 5 % as compared to its activity at the optimum pH of the lactase.

The term "activity at the optimum pH" means the lactase activity at the pH, where the lactase has its optimum activity.

The term "non-lactose carbohydrate" means any carbohydrate, which is not lactose, and which a lactose-deficient lactic acid bacterium used in the process of the invention is capable of metabolizing.

The term "depletion" in relation to non-lactose carbohydrate means that the concentration of the non-lactose carbohydrate is zero or so low so that the starter culture is no longer capable of growing.

The expression "at the start of the fermentation step" means shortly before, at the same time as or shortly after addition of the starter culture to the milk base. Here, the term "shortly" means less than 30 minutes".

The expression "during the fermentation step" means at any time during the fermentation after the start and before the end of the fermentation.

The expression "at the end of the fermentation step" means shortly before, at the same time as or shortly after the target pH is reached. Here, the term "shortly" means less than 30 minutes".

The term "target pH" means the pH at which the fermentation step ends. Depending on various parameters of the process, the fermentation step is terminated by a method selected from the group consisting of 1) acidification of the fermented milk rendering at least one strain of the starter culture unable to grow, 2) cooling treatment and 3) depletion of the non-lactose carbohydrate.

In the present context, the term "mutant strain" should be understood as strains derived, or strains which can be derived from a strain (or their mother strain) of the invention by means of e.g. genetic engineering, radiation and/or chemical treatment. The "strains derived therefrom" can also be spontaneously occurring mutants. It is preferred that the "strains derived therefrom" are functionally equivalent mutants, e.g. mutants that have substantially the same, or improved properties as their mother strain. Especially, the term "mutant strains" refers to strains obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light, or to a spontaneously occurring mutant. A mutant may have been subjected to several mutagenization treatments (a single treatment should be understood as one mutagenization step followed by a screening/selection step), but it is presently preferred that no more than 20, or no more than 10, or no more than 5, treatments (or screening/selection steps) are carried out. In a presently preferred mutant less than 1%, less than 0.1%, less than 0.01%, less than 0.001% or even less than 0.0001% of the nucleotides in the bacterial genome have been replaced with another nucleotide, or deleted, compared to the mother strain.

## DEPOSITS AND EXPERT SOLUTION

The Applicant requests that a sample of the deposited microorganism should be made available only to an expert approved by the Applicant.

*Streptococcus thermophilus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952.

*Streptococcus thermophilus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953.

*Streptococcus thermophilus* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32599.

*Streptococcus thermophilus* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32600.

*Lactococcus lactis* subsp. *cremoris* strain deposited with DSMZ-Deutsche Sammlung von Mikroorganismen und Zellkulturen GmbH, Inhoffenstr. 7B, D-38124 Braunschweig, on 2016-12-06 under the accession no. DSM 32398.

*Lactococcus lactis* subsp. *cremoris* strain deposited with DSMZ-Deutsche Sammlung von Mikroorganismen und Zellkulturen GmbH, Inhoffenstr. 7B, D-38124 Braunschweig, on 2006-12-19 under the accession no. DSM 18882.

*Lactococcus lactis* subsp. *cremoris* strain deposited with DSMZ-Deutsche Sammlung von Mikroorganismen und Zellkulturen GmbH, Inhoffenstr. 7B, D-38124 Braunschweig, on 2006-12-19 under the accession no. DSM 18893.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2016-12-06 under the accession no. DSM 32399.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32601.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32602.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32603.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32604.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2017-08-22 under the accession no. DSM 32605.

*Lactococcus lactis* subsp. *cremoris* strain deposited with DSMZ-Deutsche Sammlung von Mikroorganismen und Zellkulturen GmbH, Inhoffenstr. 7B, D-38124 Braunschweig, on 2018-06-05 under the accession no. DSM 32829.

*Lactococcus lactis* subsp. *cremoris* strain deposited with DSMZ-Deutsche Sammlung von Mikroorganismen und Zellkulturen GmbH, Inhoffenstr. 7B, D-38124 Braunschweig, on 2018-06-05 under the accession no. DSM 32830.

*Lactococcus lactis* subsp. *lactis* strain deposited with DSMZ-Deutsche Sammlung von Mikroorganismen und Zellkulturen GmbH, Inhoffenstr. 7B, D-38124 Braunschweig, on 2018-06-05 under the accession no. DSM 32832.

The deposits were made according to the Budapest treaty on the international recognition of the deposit of microorganisms for the purposes of patent procedure.

## EXAMPLES

## EXAMPLE 1

*Production of stirred sour cream using sucrose as carbohydrate source and a culture consisting of one lactose-deficient S. thermophilus (ST) and one or two lactose-deficient L. lactis* subsp. cremoris (CR)

## Strains

ST1: *Streptococcus thermophilus* DSM 28952
CR1: *Lactococcus lactis* subsp. *cremoris* DSM 32398
CR7: *Lactococcus lactis* subsp. *cremoris* DSM 18893

## Culture compositions

Table 1

|  | ST1 (%) | CR1 (%) | CR7 (%) |
|---|---|---|---|
| M1 | 72.2 | 27.8 | 0.0 |
| M2 | 72.2 | 13.9 | 13.9 |

As a basis of comparison a reference culture was used containing a conventional lactose-positive *Streptococcus thermophilus* strain and a conventional lactose-positive *Lactococcus lactis* strain.

## Milk base

The milk base for the reference culture contained 3.5 % protein and 15 % fat. The milk base for cultures of the invention contained 3.2 % protein and 15 % fat.

Table 2: Composition of milk base

|  | Milk base for invention (%) | Milk base for reference (%) |
|---|---|---|
| Milk | 50.74 | 50.63 |
| Cream | 46.87 | 46.87 |
| Skimmed Milk Powder | 1.67 | 2.49 |

(continued)

|  | Milk base for invention (%) | Milk base for reference (%) |
|---|---|---|
| Sugar (Sucrose) | 0.71 | 0.0 |

## Procedure

Fermentation was carried out at temperatures 30 °C and 34 °C.

Samples of the stirred sour cream produced were filled into cups and stored at 6 °C and pH was measured at the end of fermentation and at days 7, 14, 21, 28, 35 and 42 after the end of fermentation.

The reference sample was cooled to 16 °C before filling into cups.

The samples produced according to the invention were not cooled prior to filling into cups.

## Measurements

Post-acidification, gel firmness and shear stress were measured.

Fat and protein levels were determined using MilkoScan analysis.

Compression test (correlated to gel firmness on a spoon as evaluated by a trained sensory panel)

A back extrusion test was conducted to evaluate gel firmness. The samples were tempered to be 13°C for one hour prior to shear stress measurements. Stirring with spoon was applied to give a homogenous sample, i.e. stirring five times. Measurement was done by TA-XT plus, software Texture Expert Exceed v6.1.9.0. A cylindrical acrylic probe (Ø 40mm) penetrated the yogurt to a depth of 15mm with a speed of 2mm/s and a trigger force of 5g. The positive area was used as firmness measurement.

*Shear stress*

Seven days after incubation, the fermented milk product was brought to 13°C and manually stirred gently by means of a spoon (5 times) until homogeneity of the sample. The rheological properties of the sample were assessed on a rheometer (Anton Paar Physica Rheometer with ASC, Automatic Sample Changer, Anton Paar® GmbH, Austria) by using a bob-cup. The rheometer was set to a constant temperature of 13 °C during the time of measurement. Settings were as follows:

Holding time (to rebuild to somewhat original structure)

5 minutes without any physical stress (oscillation or rotation) applied to the sample. Oscillation step (to measure the elastic and viscous modulus, G' and G", respectively, therefore calculating the complex modulus G*)

Constant strain = 0.3 %, frequency (f) = [0.5...8] Hz
6 measuring points over 60 s (one every 10 s)

Rotation step (to measure shear stress at 300 1/s)

Two steps were designed:
1) Shear rate = [0.3-300] 1/s and 2) Shear rate = [275-0.3] 1/s.

Each step contained 21 measuring points over 210 s (on every 10 s).

The shear stress at 300 1/s was chosen for further analysis, as this correlates to mouth thickness when swallowing a fermented milk product.

## Results

*Post-acidification*

Table 3

|  | Day 0 (end pH) | Day 7 | Day 14 | Day 21 | Day 28 | Day 35 | Day 42 |
|---|---|---|---|---|---|---|---|
| Ref. at 30 °C | 4.50 | 4.46 | 4.43 | 4.41 | 4.45 | 4.43 | 4.41 |
| M1 at 30 °C | 4.48 | 4.48 | 4.47 | 4.45 | 4.45 | 4.45 | 4.45 |
| M2 at 30 °C | 4.50 | 4.52 | 4.49 | 4.49 | 4.49 | 4.49 | 4.49 |
| Ref. at 34 °C | 4.50 | 4.48 | 4.42 | 4.40 | 4.40 | 4.39 | 4.40 |
| M1 at 34 °C | 4.45 | 4.44 | 4.44 | 4.42 | 4.37 | 4.37 | 4.40 |
| M2 at 34 °C | 4.50 | 4.52 | 4.44 | 4.44 | 4.42 | 4.42 | 4.42 |

As will appear for the results of Table 3, for the samples fermented at 30 °C the post-acidification of the samples produced in accordance with the process of the invention was less than that of the reference sample. For the samples fermented at 34 °C the post-acidification of sample M2 produced in accordance with the process of the invention was less than that of the reference sample, whereas the post-acidification of sample M1 was at the same level as that of the reference sample.

*Gel firmness*

Table 4

|  | Positive area (g.s) |
|---|---|
| Ref. at 30 °C | 526.8 |
| M1 at 30 °C | 560.01 |
| M2 at 30 °C | 648.28 |
| Ref. at 34 °C | 510.58 |
| M1 at 34 °C | 639.0 |
| M2 at 34 °C | 999.55 |

As will appear from Table 4, the gel firmness of the samples produced in accordance with the process of the invention was significantly enhanced as compared to the corresponding reference samples.

*Shear stress*

Table 5

|  | Shear stress (Pa) |
|---|---|
| Ref. at 34 °C | 148 |
| M1 at 34 °C | 156 |
| M2 at 34 °C | 170 |

As will appear from Table 5, the shear stress of the samples produced in accordance with the process of the invention was significantly enhanced as compared to the reference sample.

*No cooling before filling*

As will appear from the above results the samples produced in accordance with the process of the invention had superior performance with respect to post-acidification and texture as compared to the reference sample, although the samples of the invention were not cooled before filling in contrast to the reference samples. Thus, the present results show that using the process of the invention it is possible to omit the step of cooling the fermented milk products before filling into end consumer cups.

## EXAMPLE 2

*Production of Quark using sucrose as carbohydrate source and a culture consisting of one lactose-deficient S. thermophilus (ST) and one or two lactose-deficient L. lactis subsp. cremoris (CR).*

The object of this experiment is the production of Quark with a target protein content of 7.5 %. The milk base consists of pure milk with 3.2 % protein and 0 % fat. Sucrose is added to the milk bases to be used for the cultures of the invention. No sucrose is added to the milk base for the reference culture. The strains, culture compositions, procedure and measurements are the same as in Example 1.

## Results

*Post-acidification*

Table 6

|  | Day 0 (end pH) | Day 7 | Day 14 | Day 21 | Day 28 |
|---|---|---|---|---|---|
| Ref. at 30 °C | 4.55 | 4.37 | 4.35 | 4.30 | 4.28 |
| M1 at 30 °C | 4.53 | 4.50 | 4.50 | 4.50 | 4.50 |
| M2 at 30 °C | 4.55 | 4.51 | 4.51 | 4.50 | 4.50 |
| Ref. at 34 °C | 4.54 | 4.45 | 4.43 | 4.40 | 4.39 |
| M1 at 34 °C | 4.47 | 4.50 | 4.47 | 4.47 | 4.47 |
| M2 at 34 °C | 4.53 | 4.50 | 4.48 | 4.48 | 4.48 |

As will appear for the results of Table 6, the post-acidification of the samples produced in accordance with the process of the invention was less than that of the corresponding reference samples.

*Gel firmness*

Table 7

|  | Positive area (g.s) |
|---|---|
| Ref. at 30 °C | 264.12 |
| M1 at 30 °C | 283.21 |
| M2 at 30 °C | 415.88 |
| Ref. at 34 °C | 113.46 |
| M1 at 34 °C | 127.71 |
| M2 at 34 °C | 126.97 |

As will appear from Table 7, the gel firmness of the samples produced in accordance with the process of the invention was significantly enhanced as compared to the corresponding reference samples.

## EXAMPLE 3

*Production of stirred sour cream using glucose as carbohydrate source and a culture consisting of one lactose-deficient S. thermophilus (ST) and one or two lactose-deficient L. lactis subsp. cremoris (CR)*

In this experiment glucose was used as carbohydrate source. Fermentation temperatures were 30 °C and 35 °C. The procedure and measurements were the same as in Example 1.

### Strains

ST1: *Streptococcus thermophilus* DSM 28952
CR1: *Lactococcus lactis* subsp. *cremoris* DSM 32398
CR2: *Lactococcus lactis* subsp. *cremoris* DSM 18882
CR7: *Lactococcus lactis* subsp. *cremoris* DSM 18893

### Culture compositions

Table 8

|      | ST1 (%) | CR1 (%) | CR2 (%) | CR7 (%) |
|------|---------|---------|---------|---------|
| M1.2 | 72.2    | 13.9    | 13.9    | 0.0     |
| M1.7 | 72.2    | 13.9    | 0.0     | 13.9    |

As a basis of comparison a reference culture was used containing a conventional lactose-positive *Streptococcus thermophilus* strain and a conventional lactose-positive *Lactococcus lactis* strain.

### Milk base

The milk base for the reference culture contained 3.5 % protein and 15 % fat. The milk base for cultures of the invention contained 3.2 % protein and 15 % fat.

Table 9: Composition of milk base

|                    | Milk base for invention (kg) | Milk base for reference (kg) |
|--------------------|------------------------------|------------------------------|
| Milk               | 18.34                        | 19.66                        |
| Cream              | 16.40                        | 17.34                        |
| Skimmed Milk Powder | 0.025                       | 0.002                        |
| Sugar (Glucose)    | 0.228                        | 0.0                          |

### Results

*Post-acidification*

Table 10

|                | Day 0 (end pH) | Day 7 | Day 21 | Day 28 |
|----------------|----------------|-------|--------|--------|
| Ref. at 30 °C  | 4.57           | 4.47  | 4.46   | 4.46   |
| M1.2 at 30 °C  | 4.54           | 4.58  | 4.64   | 4.62   |
| M1.7 at 30 °C  | 4.58           | 4.62  | 4.68   | 4.65   |

(continued)

|  | Day 0 (end pH) | Day 7 | Day 21 | Day 28 |
|---|---|---|---|---|
| Ref. at 35 °C | 4.57 | 4.48 | 4.50 | 4.47 |
| M1.2 at 35 °C | 4.58 | 4.60 | 4.65 | 4.62 |
| M1.7 at 35 °C | 4.56 | 4.60 | 4.66 | 4.64 |

As will appear for the results of Table 10, the post-acidification of the samples produced in accordance with the process of the invention was less than that of the corresponding reference samples.

*Gel firmness*

Table 11

|  | Positive area (g.s) |
|---|---|
| Ref. at 30 °C | 1650.46 |
| M1.2 at 30 °C | 1718.41 |
| M1.7 at 30 °C | 1658.08 |
| Ref. at 35 °C | 1880.56 |
| M1.2 at 35 °C | 2494.80 |
| M1.7 at 35 °C | 1970.33 |

As will appear from Table 11, the gel firmness of the samples produced in accordance with the process of the invention was significantly enhanced as compared to the corresponding reference samples.

## EXAMPLE 4

*Production of quark using glucose as carbohydrate source and a culture consisting of one lactose-deficient S. thermophilus (ST) and one or two lactose-deficient L. lactis subsp. cremoris (CR)*

In this experiment glucose was used as carbohydrate source. Fermentation temperature was 30 °C. The strains, culture compositions, procedure and measurements are the same as in Example 3.

## Milk base

Table 12

|  | Milk base for invention (kg) |
|---|---|
| Milk | 59.61 |
| Glucose | 0.390 |

## Results

*Post-acidification*

Table 13

| | Day 0 (end pH) | Day 7 | Day 14 | Day 21 | Day 28 | Day 36 | Day 42 | Day 49 |
|---|---|---|---|---|---|---|---|---|
| Ref. at 30 °C | 4.63 | 4.45 | 4.39 | 4.37 | 4.35 | 4.38 | 4.38 | 4.35 |
| M1.2 at 30 °C | 4.68 | 4.78 | 4.78 | 4.78 | 4.74 | 4.80 | 4.80 | 4.74 |
| M1.7 at 30 °C | 4.65 | 4.82 | 4.78 | 4.78 | 4.78 | 4.78 | 4.77 | 4.76 |

As will appear for the results of Table 13, the post-acidification of the samples produced in accordance with the process of the invention was less than that of the corresponding reference samples.

*Gel firmness*

Table 14

| | Positive area (g.s) |
|---|---|
| Ref. at 30 °C | 88.2 |
| M1.2 at 30 °C | 104.53 |
| M1.7 at 30 °C | 132.44 |

As will appear from Table 14, the gel firmness of the samples produced in accordance with the process of the invention was significantly enhanced as compared to the corresponding reference samples.

## EXAMPLE 5

*Production of quark using glucose and sucrose as carbohydrate source and a culture consisting of one lactose-deficient S. thermophilus (ST) and two lactose-deficient L. lactis subsp. cremoris (CR)*

The milk based consisted of skim milk containing either 0.7 % glucose or 0.6 % sucrose. Fermentation was carried out at 30 °C until a target pH of 4.6 was reached. Samples were stirred and cooled in ice water for approx. 15 minutes and then stored at 5 °C. Texture (shear stress) was measured at day 7.

## Strains

ST2: *Streptococcus thermophilus* DSM 32599.

## Culture compositions

Table 15

| | ST1 (%) | ST2 (%) | CR1 (%) | CR7 (%) |
|---|---|---|---|---|
| M1.7_ST1 | 72.2 | | 13.9 | 13.9 |
| M1.7_ST2 | | 72.2 | 13.9 | 13.9 |

## Results

Shear stress

Table 16

|  | Carbohydrate | Shear stress (Pa) |
|---|---|---|
| M1.7_ST1 | Glucose | 30.55 |
| M1.7_ST2 | Glucose | 31.15 |
| M1.7_ST1 | Sucrose | 42.00 |
| M1.7_ST2 | Sucrose | 39.40 |

As will appear from Table 16, cultures with the two *Streptococcus thermophilus* produced quark with the same level of shear stress. Also, the samples grown on sucrose had a higher level of shear stress than the samples grown on glucose.

**EXAMPLE 6**

*Acidification profiles of cultures consisting of one lactose-deficient S. thermophilus (ST) and one or two lactose-deficient, sucrose-positive L. lactis subsp. lactis (LC)*

**Strains**

ST1: *Streptococcus thermophilus* DSM 28952
ST2: *Streptococcus thermophilus* DSM 32599
LC1: *Lactococcus lactis* subsp. *lactis* DSM 32603
LC2: *Lactococcus lactis* subsp. *lactis* DSM 32604
LC3: *Lactococcus lactis* subsp. *lactis* DSM 32601
LC4: *Lactococcus lactis* subsp. *lactis* DSM 32602
LC5: *Lactococcus lactis* subsp. *lactis* DSM 32605

**Culture compositions**

Table 17

|  | ST (%) | ST2 (%) | LC1 (%) | LC2 (%) | LC3 (%) | LC4 (%) | LC5 (%) |
|---|---|---|---|---|---|---|---|
| C1 | 72.2 |  | 27.8 |  |  |  |  |
| C2 | 72.2 |  |  | 27.8 |  |  |  |
| C3 | 72.2 |  |  |  | 27.8 |  |  |
| C4 | 72.2 |  |  |  |  | 27.8 |  |
| C5 | 72.2 |  |  |  |  |  | 27.8 |
| C6 |  | 72.2 | 27.8 |  |  |  |  |

As a basis of comparison a reference culture was used containing a conventional lactose-positive *Streptococcus thermophilus* strain and a conventional lactose-positive *Lactococcus lactis* strain.

**Procedure**

Overnight cultures grown in M17 medium containing 1 % sucrose were inoculated in 200 ml B-milk containing 0.5 % sucrose and 0.02 g/L Na-format and incubated in water bath at either 30 °C or 34 °C. pH is followed for an extended period of time after the target pH is reached.

### Results

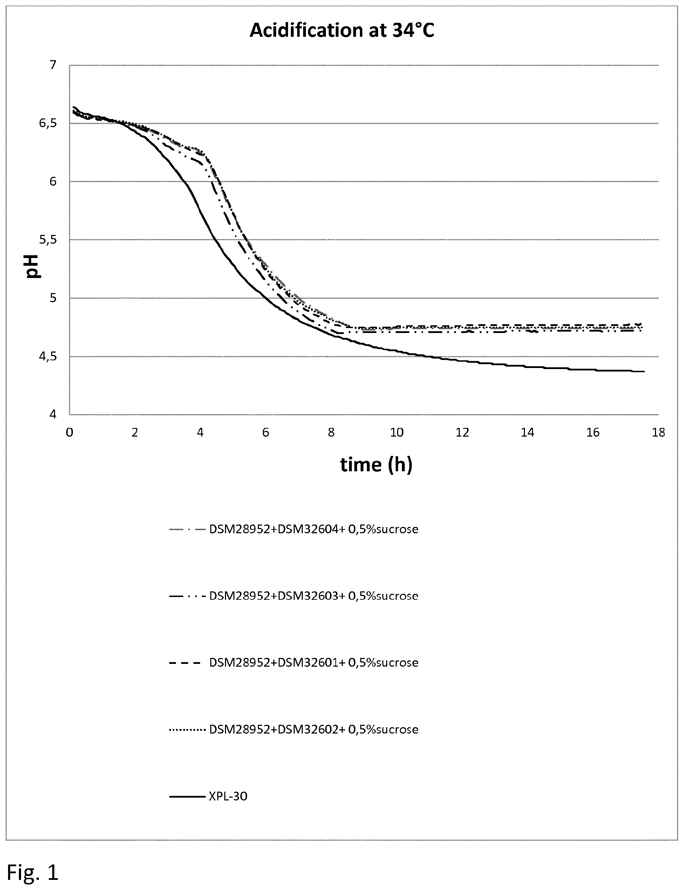

Fig. 1 shows the acidification profiles of C1-C4 at 34 °C.

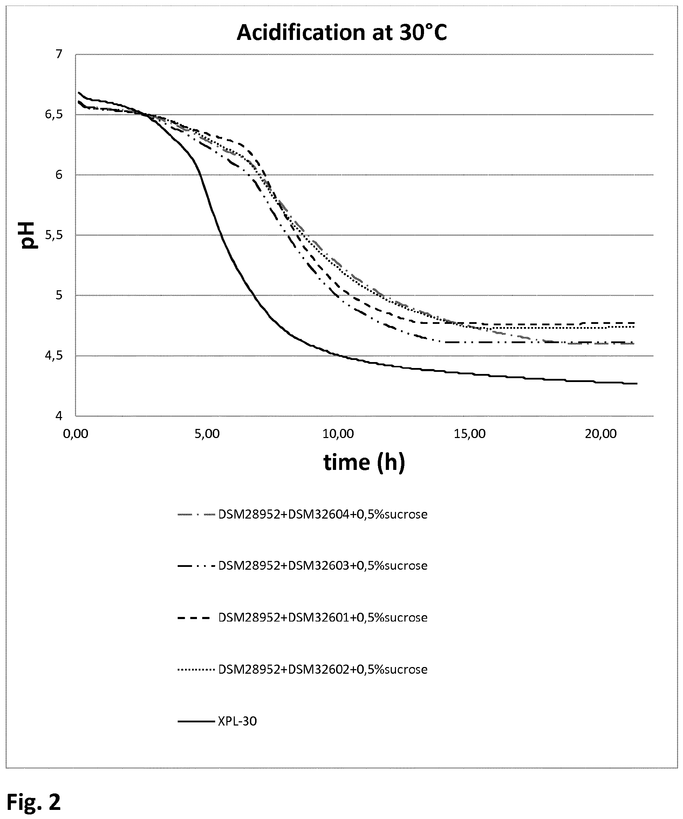

Fig. 2 shows the acidification profiles of C1-C4 at 30 °C.

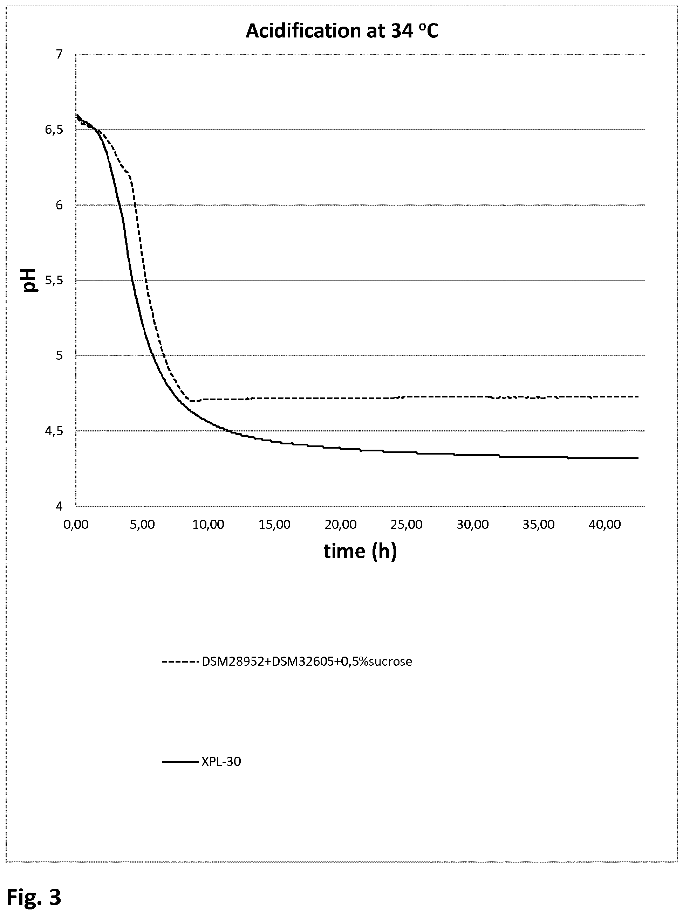

Fig. 3 shows the acidification profile of C5 at 34 °C.

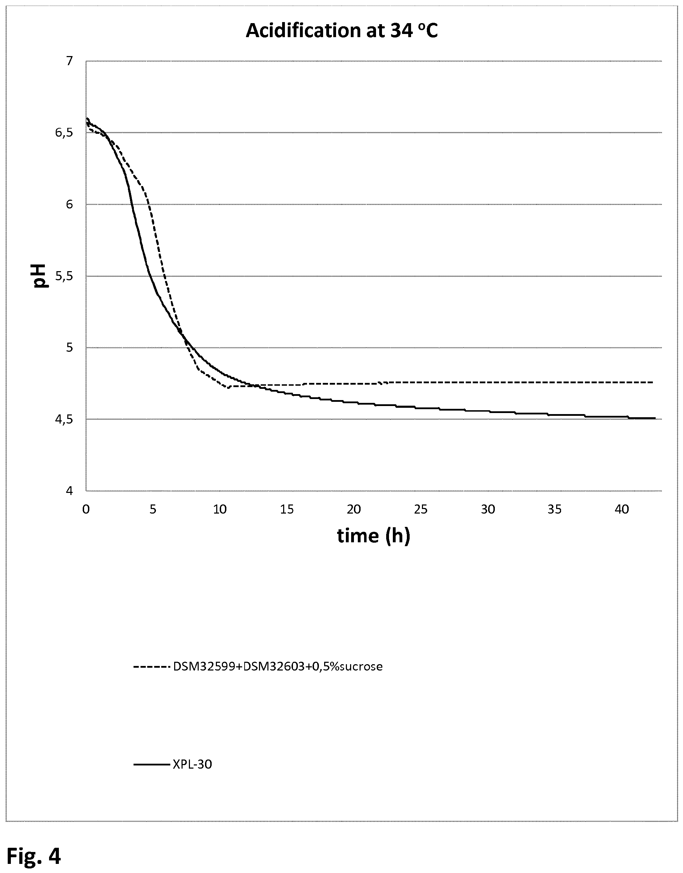

Fig. 4 shows the acidification profile of C6 at 34 °C.

As will appear from Fig. 1 and 3 for all of cultures C1-C5 at 34 °C the pH drops to a target level of approx. 4.7- 4.8 in approx. 8 hours. As will appear from Fig. 2 for cultures C1-C4 at 30 °C the pH drops to a target level of approx. 4.7- 4.8 in approx. 14 hours. As will appear from Fig. 4 for culture C6 at 34 °C the pH drops to a target level of approx. 4.7- 4.8 in approx. 10 hours. After reaching the target pH the pH remains completely constant for the following period as long as pH is measured, i.e. no post-acidification occurs.

In comparison, for the reference product the pH continues to decline for the entire period wherein pH is followed, i.e. post-acidification occurs.

### EXAMPLE 7

*Acidification profiles of two lactose-deficient, sucrose positive Lactococcus lactis subsp. lactis (LC) strains and two lactose-deficient, glucose positive Lactococcus lactis subsp. lactis cremoris (LACcr) strains.*

### Strains

LC5: *Lactococcus lactis* subsp. *lactis* DSM 32605

LC7: *Lactococcus lactis* subsp. *lactis* DSM 32832

LACcr1: *Lactococcus lactis* subsp. *lactis* DSM 32829

LACcr2: *Lactococcus lactis* subsp. *lactis* DSM 32830

ST1: *Streptococcus thermophilus* DSM 28952

### Culture compositions

LC5 + ST1

LC7 + ST1

LACcr1 + ST1

LACcr2 + ST1

### Procedure

Overnight cultures of LC5 and LC7 were grown in autoclaved milk (A-milk) containing 2 % and used to inoculate 200 ml skim milk containing 0.5 % sucrose.

ST1 was inoculated with 0.0065%. Fermentation was carried out at 30 °C for 24 hours

LACcr1 and LACcr2 strains were inoculated directly from Pre-Inoculation Material (PIM) corresponding to 1.1E+09 cells / 200 ml milk. ST1 was inoculated with 0.0065%. Fermentation was carried out in skim milk containing 0.5 % glucose at 30 °C for 47 hours

### Results

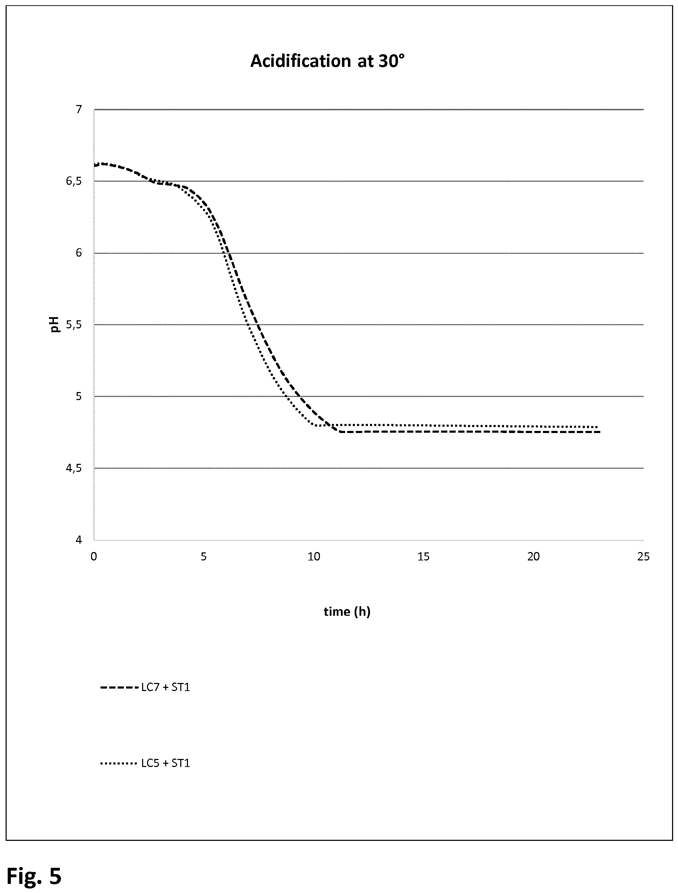

Fig. 5 shows the acidification profiles of LC5 + ST1 and LC7 + ST1 at 30 °C.

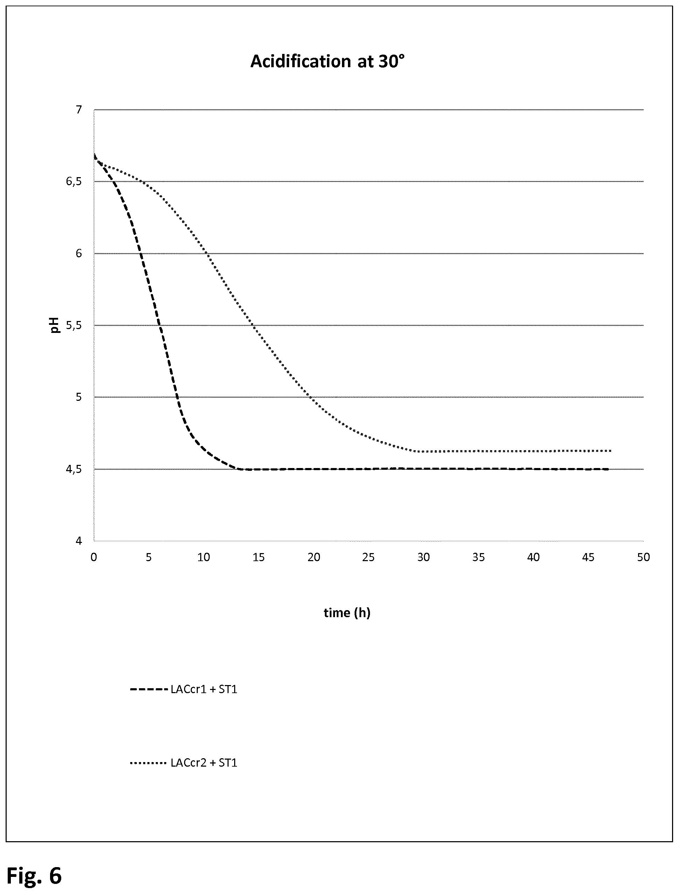

Fig. 6 shows the acidification profiles of LACcr1 + ST1 and LACcr2 + ST1 at 30 °C.

As will appear from Fig. 5 and 6 the pH drops to a stable level reflecting a termination of the fermentation due to decrease/depletion of the added carbohydrate source. After reaching the stable pH it remains completely constant for the following period for as long as pH is measured, i.e. no post-acidification occurs.

### EXAMPLE 8

*Acidification profiles and post acidification of culture blends consisting of one lactose-deficient, sucrose positive*

*Lactococcus lactis subsp lactis (LC) strains, one lactose-deficient, glucose positive Lactococcus lactis subsp cremoris (LACcr), one lactose-deficient S. thermophilus (ST) and one mild S. thermophilus.*

### Strains

LC5: *Lactococcus lactis* subsp. *lactis* DSM 32605
LC7: *Lactococcus lactis* subsp. *lactis* DSM 32832
LACcr1: *Lactococcus lactis* subsp. *cremoris* DSM 32829
LACcr2: *Lactococcus lactis* subsp. *cremoris* DSM 32830
ST1: *Streptococcus thermophilus* DSM 28952
STmild: Commercial *Streptococcus thermophilus* strain with a low acidification capacity.

### Culture compositions

Table 18

|    | ST1 | ST mild | LC5 | LC7 | LACcr1 | LACcr |
|----|-----|---------|-----|-----|--------|-------|
| C1 | X   | X       | X   |     | X      |       |
| C2 | X   | X       | X   |     |        | X     |
| C3 | X   | X       |     | X   | X      |       |
| C4 | X   | X       |     | X   |        | X     |

As a basis of comparison, a reference culture was used containing a conventional lactose-positive *Streptococcus thermophilus* strain and a conventional lactose-positive *Lactococcus lactis* strain.

### Procedure

Culture blends C1-C4 were acidified in skim milk containing 0.6% sucrose. Fermentation was carried out at 30 °C for 18 hours. Samples were stirred and cooled in ice water for approx. 15 minutes and then stored at 5 °C. Post-acidification (pH) was measured after a cold storage period of 28 days and shear stress was measured at day 7 using the method described in Example 1.

### Results

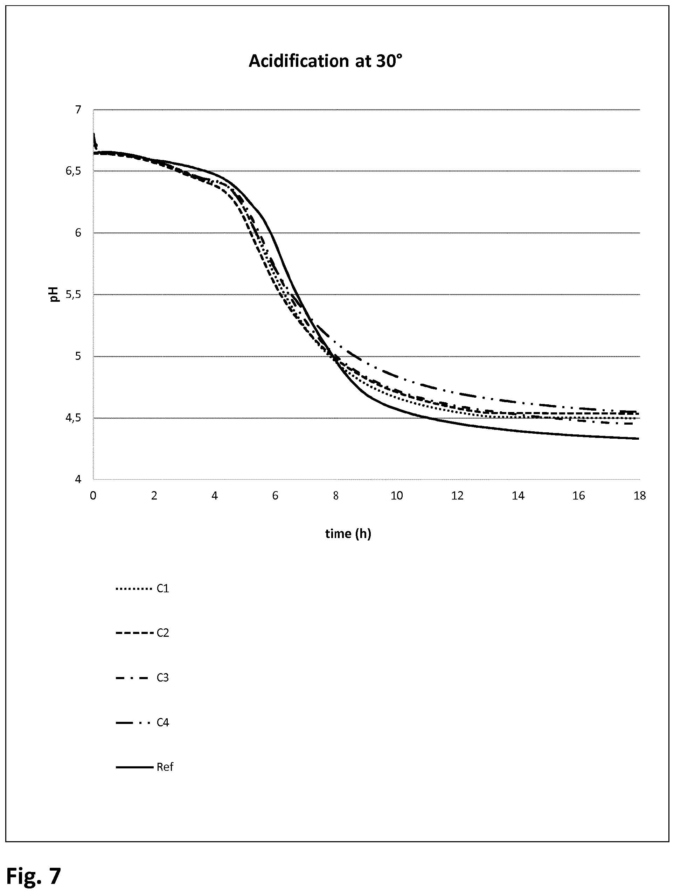

Fig. 7 shows the acidification profiles of C1- C4 and the reference at 30 °C.

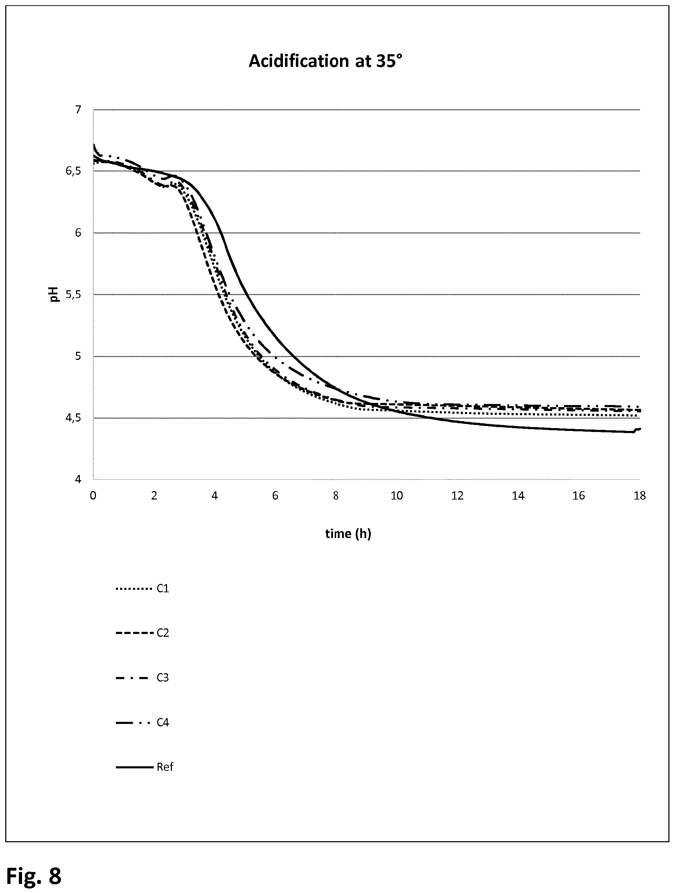

Fig. 8 shows the acidification profiles of C1- C4 and the reference at 35 °C.
As will appear from the Fig 7 (30°C) the pH stabilizes at 4.55 for the culture blends C1-C3 after approximately 13 hours and for C4 after approximately 17 hours. Acidification profiles at 35° (Fig. 8) show pH stabilization at approximately pH 4.60 after 11 hours for all culture blends C1-C4. For the reference culture, the pH continues to drop throughout the full period monitored (18 hours) at both 30°C and 35°C.

Table 19: Long term post-acidification and shear stress

|     | Temperature | pH Day 28 | Shear stress at 300 1/s |
|-----|-------------|-----------|-------------------------|
| C1  |             | 4.45      | 39.90                   |
| C2  |             | 4.53      | 43.45                   |
| C3  | 30°C        | 4.44      | 41.65                   |
| C4  |             | 4.48      | 48.00                   |
| Ref |             | 4.31      | 40.35                   |

(continued)

|  | Temperature | pH Day 28 | Shear stress at 300 1/s |
|---|---|---|---|
| C1 |  | 4.43 | 46.80 |
| C2 |  | 4.54 | 47.45 |
| C3 | 35°C | 4.52 | 50.75 |
| C4 |  | 4.52 | 48.65 |
| Ref |  | 4.38 | 40.85 |

As will appear from Table 19, the pH values of the culture blends C1-C4 stored for 28 days at 30°C are between 0.13-0.22 pH units higher than the pH of the reference culture. The pH values of the culture blends C1-C4 stored for 28 days at 35°C are between 0.05-0.16 pH units higher than the reference.

For both the samples stored 30°C and at 35°C, the shear stress was either at the same level or higher for the culture blends C1-C4 than the shear stress for the reference culture.

## EXAMPLE 9

*Production of stirred sour cream using sucrose as carbohydrate source and a culture consisting one lactose-deficient, sucrose positive Lactococcus lactis subsp lactis (LC) strain, one lactose-deficient, glucose positive Lactococcus lactis subsp cremoris (CR / LACcr, one lactose-deficient S. thermophilus (ST) and one mild S. thermophilus (STmild).*

## Strains

LC7: *Lactococcus lactis* subsp. *lactis* DSM 32832
LACcr1: *Lactococcus lactis* subsp. *cremoris* DSM 32829
LACcr2: *Lactococcus lactis* subsp. *cremoris* DSM 32830
CR7: *Lactococcus lactis* subsp. *cremoris* DSM 18893
ST1: *Streptococcus thermophilus* DSM 28952
STmild1: Commercial *Streptococcus thermophilus* strain with a low acidification capacity.
STmild2: Commercial *Streptococcus thermophilus* strain with a low acidification capacity.

## Culture compositions

Table 20

|  | ST1 | STmild 1 | STmild2 | LC7 | LACcr1 | LACcr2 | CR7 |
|---|---|---|---|---|---|---|---|
| C1 | X |  | X | X | X |  |  |
| C2 | X | X |  | X |  | X |  |
| C3 | X |  | X | X |  |  | X |

As a basis of comparison, a reference culture was used containing a conventional lactose-positive *Streptococcus thermophilus* strain and a conventional lactose-positive *Lactococcus lactis* strain.

## Milk base

The milk base for the reference culture contained 2.7 % protein and 15 % fat. The milk base for cultures of the invention contained 2.4 % protein, 15 % fat and 0.45% sucrose.

Table 21: Composition of milk base

|  | Milk base for invention (%) | Milk base for reference (%) |
| --- | --- | --- |
| Milk | 46.00 | 52.96 |
| Cream | 46.87 | 46.88 |
| Skimmed Milk Powder |  | 0.16 |
| Sugar (Sucrose) | 0.45 |  |
| Water | 6.68 |  |
| Treatment | Homogenization: 200/40 bar at 70°C<br>Heat treatment: 92°C / 6 min | |

## Procedure

Fermentation was carried out at temperatures 30 °C and 35 °C.

Reference was post treated using 2 bar back pressure and cooling to 18°C. The samples produced according to the invention were post treated using 2 bar back pressure at fermentation temperature (30°c and 35°C).

Samples were stored at 6 °C.

Post acidification (pH) was measured after a cold storage period of 28 days and gel firmness was measured after day 7. The procedures for texture measurements (gel firmness) were the same as in Example 1.

## Measurements

Post-acidification and gel firmness were measured.

Table 22: Post-acidification

| pH | Day 0 (end pH) | Day 1 | Day 7 | Day 14 | Day 28 | Day 1 minus Day 35 |
| --- | --- | --- | --- | --- | --- | --- |
| Ref. at 30°C | 4.55 | 4.53 | 4.5 | 4.45 | 4.41 | -0.12 |
| C1 at 30°C | 4.48 | 4.48 | 4.48 | 4.49 | 4.46 | -0.02 |
| C2 at 30°C | 4.53 | 4.52 | 4.52 | 4.52 | 4.52 | -0.00 |
| C3 at 30°C | 4.44 | 4.44 | 4.43 | 4.42 | 4.41 | -0.03 |
| Ref. at 35°C | 4.55 | 4.54 | 4.52 | 4.50 | 4.46 | -0.08 |
| C1 at 35°C | 4.56 | 4.55 | 4.53 | 4.52 | 4.49 | -0.06 |
| C2 at 35°C | 4.51 | 4.5 | 4.49 | 4.48 | 4.46 | -0.04 |
| C3 at 35°C | 4.56 | 4.55 | 4.52 | 4.52 | 4.49 | -0.06 |

As will appear from the results of Table 22, the post-acidification of the samples produced in accordance with the process of the invention was less than that of the corresponding reference samples.

Table 23: Gel firmness

|  | Gel firmness Positive area (g.s) |
| --- | --- |
| Ref. at 30°C | 1299 |
| C1 at 30°C | 2164 |
| C2 at 30°C | 2789 |
| C3 at 30°C | 2832 |
| Ref. at 35°C | 1272 |
| C1 at 35°C | 2885 |
| C2 at 35°C | 2684 |

(continued)

| | Gel firmness Positive area (g.s) |
|---|---|
| C3 at 35°C | 3130 |

As will appear from Table 23, the gel firmness of the samples produced in accordance with the process of the invention was significantly enhanced as compared to the corresponding reference samples.

## EXAMPLE 10

*Production of stirred quark using sucrose as carbohydrate source and a culture consisting one lactose-deficient, sucrose positive Lactococcus lactis subsp lactis (LC) strain, one lactose-deficient, glucose positive Lactococcus lactis subsp cremoris (CR / LACcr, one lactose-deficient S. thermophilus (ST) and one mild S. thermophilus (STmild). The strains and culture compositions, are the same as in Example 9.*

### Milk base

Skimmed milk was used as milk base.

The milk base for the reference culture contained 3.2 % protein and 0.05 % fat. The milk base for cultures of the invention contained 3.2 % protein, 0.05 % fat and sucrose levels between 0.45% and 0.65% selected so as to be optimized to each specific culture composition.

### Procedure

Fermentation was carried out at temperatures 30 °C and 35 °C. Culture inoculation percentage was 0.01%.

Samples were stored at 6°C.

### Results

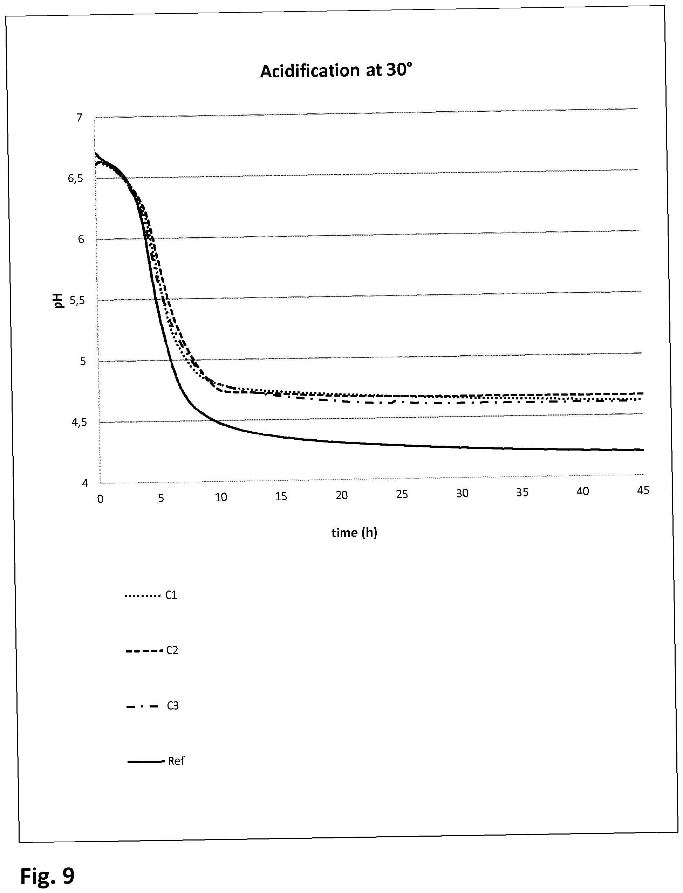

Fig 9 shows the acidification profiles of C1- C3 and the reference at 30 °C.

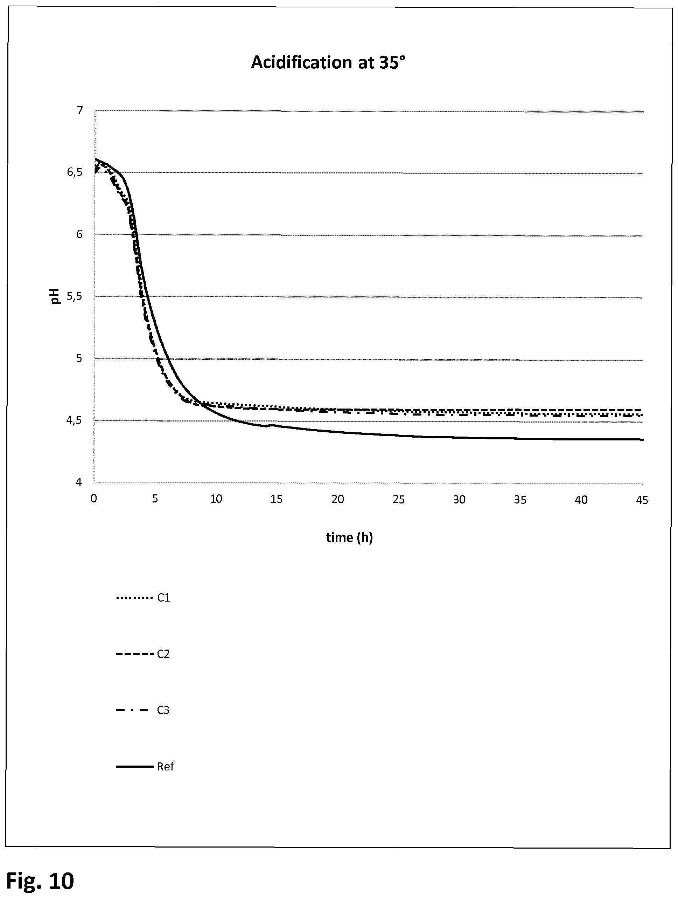

Fig 10 shows the acidification profiles of C1- C3 and the reference at 35 °C

As will appear from the Fig 9 (30°C) the pH stabilizes for the culture blends C1-C3 after approximately 12 hours. Acidification profiles at 35° (Fig. 10) show pH stabilization after approximately 11 hours of fermentation. For the reference culture, the pH drops to a significant lower value during the period monitored at both 30°C and 35°C.

Post-acidification was measured at different time periods of cold storage (6°C).

Table 24: Post-acidification

| pH | Day 0 (end pH) | Day 3 | Day 8 | Day 9 | Day 13 |
|---|---|---|---|---|---|
| Ref. at 30°C | 4.68 | ND | 4.37 | ND | 4.38 |
| C1 at 30°C | 4.71 | ND | 4.70 | ND | 4.70 |
| C2 at 30°C | 4.68 | ND | 4.73 | ND | 4.73 |
| C3 at 30°C | 4.64 | ND | 4.68 | ND | 4.69 |
| Ref. at 35°C | 4.60 | 4.47 | ND | 4.48 | ND |
| C1 at 35°C | 4.60 | 4.60 | ND | 4.60 | ND |
| C2 at 35°C | 4.59 | 4.62 | ND | 4.61 | ND |
| C3 at 35°C | 4.58 | 4.59 | ND | 4.59 | ND |
| ND: No data. | | | | | |

As will appear from the results of Table 24, the post-acidification of the samples produced in accordance with the process of the invention was less than that of the corresponding reference samples.

Table 25: Gel firmness

|  | Gel firmness Positive area (g.s) |
|---|---|
| Ref. at 30°C | 594.96 |
| C1 at 30°C | 744.85 |
| C2 at 30°C | 578.39 |
| C3 at 30°C | 712.14 |

As will appear from Table 25, the gel firmness of the samples produced in accordance with the process of the invention at 30°C was significantly enhanced as compared to the corresponding reference sample.

**Claims**

1.   A process for producing a fermented milk product comprising the steps of

1) adding a starter culture of lactic acid bacteria comprising at least one lactose-deficient *Streptococcus thermophilus* strain, which is capable of metabolizing a non-lactose carbohydrate, and at least one lactose-deficient *Lactococcus lactis* strain, which is capable of metabolizing the non-lactose carbohydrate, to a milk base, and
2) fermenting the milk for a period of time until a target pH is reached to obtain a fermented milk product,

wherein non-lactose carbohydrate is added to the milk base at the start of the fermentation step.

2.   A process according to claim 1, wherein the lactose-deficient strains are capable of metabolizing a non-lactose carbohydrate selected from the group consisting of sucrose, galactose and glucose.

3.   A process according to claim 1 or 2, wherein the non-lactose carbohydrate is added to the milk base in an amount measured so as to become depleted at the target pH between 3.2 and 4.8 and hence result in stopping the growth of the lactic acid bacteria and in stopping the fermentation.

4.   A process according to any of the preceding claims, wherein the *Streptococcus thermophilus* lactose-deficient strain is selected from the group consisting of:

(a) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952;
(b) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953;
(c) the strain deposited with DSMZ-Deutsche Sammlung von Mikroorganismen und Zellkulturen GmbH, Inhoffenstr. 7B, D-38124 Braunschweig, on 2017-08-22 under the accession no. DSM 32599; and
(d) the strain deposited with DSMZ-Deutsche Sammlung von Mikroorganismen und Zellkulturen GmbH, Inhoffenstr. 7B, D-38124 Braunschweig, on 2017-08-22 under the accession no. DSM 32600.

5.   A process according to any of the preceding claims, wherein the *Lactococcus lactis* strain is selected from the group consisting of a *Lactococcus lactis* subsp. *cremoris* strain and a *Lactococcus lactis* subsp. *lactis* strain.

6.   A process according to any of the preceding claims, wherein the starter culture further contains one or more strains selected from the group consisting of *Lactococcus lactis* subsp. *lactis* biovar. *diacetylactis, Leuconostoc spp.* and *Bifidobacterium spp.*

7.   A process according to any of the preceding claims, wherein the fermented milk product produced is selected from the group consisting of buttermilk, sour milk, cultured milk, Smetana, sour cream, thick cream, cultured cream, ymer, fermented whey, Kefir, and fresh cheese, such as Quark, tvarog and cream cheese.

8.   A process according to any of the preceding claims, wherein the lactose-deficient *Lactococcus lactis* strain is selected from the group consisting of

1) the strain deposited at DSMZ under the accession number DSM 32398,
2) the strain deposited at DSMZ under the accession number DSM 18882,
3) the strain deposited at DSMZ under the accession number DSM 32399,
4) the strain deposited at DSMZ under the accession number DSM 18893,
5) the strain deposited at DSMZ under the accession number DSM 32601,
6) the strain deposited at DSMZ under the accession number DSM 32602,
7) the strain deposited at DSMZ under the accession number DSM 32603,
8) the strain deposited at DSMZ under the accession number DSM 32604,
9) the strain deposited at DSMZ under the accession number DSM 32605,
10) the strain deposited at DSMZ under the accession number DSM 32829,
11) the strain deposited at DSMZ under the accession number DSM 32830, and
12) the strain deposited at DSMZ under the accession number DSM 32832.

9. A composition of lactic acid bacteria comprising at least one lactose-deficient *Streptococcus thermophilus* strain and at least one lactose-deficient *Lactococcus lactis.*

10. A fermented milk product produced by the process of claims 1-8.

11. Use in a process for producing a fermented milk product comprising the steps of

1) adding a starter culture of lactic acid bacteria strain to a milk base, and
2) fermenting the milk for a period of time until a target pH is reached to obtain a fermented milk product, of a starter culture of lactic acid bacteria comprising at least one lactose-deficient *Streptococcus thermophilus* strain, which is capable of metabolizing a non-lactose carbohydrate, and at least one lactose-deficient *Lactococcus lactis* strain, which is capable of metabolizing the non-lactose carbohydrate.

12. Use according to claim 11 to increase the texture measured as shear stress or gel firmness of the fermented milk product as compared to using a starter culture comprising at least one lactose-positive *Streptococcus thermophilus* strain, which is capable of metabolizing lactose, and at least one lactose-positive *Lactococcus lactis* strain, which is capable of metabolizing lactose.

## Patentansprüche

1. Verfahren zum Herstellen eines fermentierten Milchproduktes, umfassend die Schritte:

1) Zusetzen einer Starterkultur von Milchsäurebakterien, umfassend mindestens einen Lactose-defizienten *Streptococcus thermophilus-Stamm,* der in der Lage ist, ein Nicht-Lactose-Kohlenhydrat zu verstoffwechseln, und mindestens einen Lactose-defizienten *Lactococcus lactis-Stamm,* der in der Lage ist, das Nicht-Lactose-Kohlenhydrat zu verstoffwechseln, zu einem Milchgrundstoff und
2) Fermentieren der Milch für eine Zeitspanne bis zum Erreichen eines Ziel-pH-Wertes, um ein fermentiertes Milchprodukt zu erhalten,

wobei dem Milchgrundstoff zu Beginn des Fermentationsschrittes Nicht-Lactose-Kohlenhydrat zugesetzt wird.

2. Verfahren nach Anspruch 1, wobei die Lactose-defizienten Stämme in der Lage sind, ein Nicht-Lactose-Kohlenhydrat, ausgewählt aus der Gruppe bestehend aus Saccharose, Galactose und Glucose, zu verstoffwechseln.

3. Verfahren nach Anspruch 1 oder 2, wobei das Nicht-Lactose-Kohlenhydrat dem Milchgrundstoff in einer Menge zugesetzt wird, die so gemessen ist, dass es bei dem Ziel-pH-Wert zwischen 3,2 und 4,8 aufgebraucht wird und somit zum Stoppen des Wachstums der Milchsäurebakterien und zum Stoppen der Fermentation führt.

4. Verfahren nach einem der vorhergehenden Ansprüche, wobei der Lactose-defiziente *Streptococcus thermophilus-Stamm* ausgewählt ist aus der Gruppe bestehend aus:

(a) dem Stamm, der bei der DSMZ-Deutsche Sammlung von Mikroorganismen und Zellkulturen GmbH, Inhoffenstr. 7B, D-38124 Braunschweig, am 12.06.2014 unter der Zugangsnr. DSM 28952 hinterlegt wurde;
(b) dem Stamm, der bei der DSMZ-Deutsche Sammlung von Mikroorganismen und Zellkulturen GmbH, Inhoffenstr. 7B, D-38124 Braunschweig, am 12.06.2014 unter der Zugangsnr. DSM 28953 hinterlegt wurde;

(c) dem Stamm, der bei der DSMZ-Deutsche Sammlung von Mikroorganismen und Zellkulturen GmbH, Inhoffenstr. 7B, D-38124 Braunschweig, am 22.08.2017 unter der Zugangsnr. DSM 32599 hinterlegt wurde;

(d) dem Stamm, der bei der DSMZ-Deutsche Sammlung von Mikroorganismen und Zellkulturen GmbH, Inhoffenstr. 7B, D-38124 Braunschweig, am 22.08.2017 unter der Zugangsnr. DSM 32600 hinterlegt wurde;

5. Verfahren nach einem der vorhergehenden Ansprüche, wobei der *Lactococcus* lactis-Stamm aus der Gruppe bestehend aus einem *Lactococcus lactis* subsp. *cremoris-Stamm* und einem *Lactococcus lactis* subsp. lactis-Stamm ausgewählt ist.

6. Verfahren nach einem der vorhergehenden Ansprüche, wobei die Starterkultur ferner einen oder mehrere Stämme enthält, der oder die aus der Gruppe bestehend aus *Lactococcus lactis* subsp. lactis biovar. *diacetylactis, Leuconostoc spp.* und *Bifidobacterium spp* ausgewählt ist oder sind.

7. Verfahren nach einem der vorhergehenden Ansprüche, wobei das fermentierte Milchprodukt aus der Gruppe bestehend aus Buttermilch, Sauermilch, kultivierter Milch, Smetana, Sauerrahm, dickem Rahm, kultiviertem Rahm, Ymer, fermentierter Molke, Kefir und Frischkäse, wie etwa Quark, Tvarog und Rahmkäse ausgewählt ist.

8. Verfahren nach einem der vorhergehenden Ansprüche, wobei der Lactose-defiziente *Lactococcus* lactis-Stamm ausgewählt ist aus der Gruppe bestehend aus:

1) dem bei der DSMZ unter der Zugangsnummer DSM 32398 hinterlegten Stamm,
2) dem bei der DSMZ unter der Zugangsnummer DSM 18882 hinterlegten Stamm,
3) dem bei der DSMZ unter der Zugangsnummer DSM 32399 hinterlegten Stamm,
4) dem bei der DSMZ unter der Zugangsnummer DSM 18893 hinterlegten Stamm,
5) dem bei der DSMZ unter der Zugangsnummer DSM 32601 hinterlegten Stamm,
6) dem bei der DSMZ unter der Zugangsnummer DSM 32602 hinterlegten Stamm,
7) dem bei der DSMZ unter der Zugangsnummer DSM 32603 hinterlegten Stamm,
8) dem bei der DSMZ unter der Zugangsnummer DSM 32604 hinterlegten Stamm,
9) dem bei der DSMZ unter der Zugangsnummer DSM 32605 hinterlegten Stamm,
10) dem bei der DSMZ unter der Zugangsnummer DSM 32829 hinterlegten Stamm,
11) dem bei der DSMZ unter der Zugangsnummer DSM 32830 hinterlegten Stamm und
12) dem bei der DSMZ unter der Zugangsnummer DSM 32832 hinterlegten Stamm.

9. Zusammensetzung von Milchsäurebakterien, umfassend mindestens einen Lactose-defizienten *Streptococcus thermophilus-Stamm* und mindestens einen Lactose-defizienten *Lactococcus lactis.*

10. Fermentiertes Milchprodukt, hergestellt durch das Verfahren der Ansprüche 1-8.

11. Verwendung in einem Verfahren zum Herstellen eines fermentierten Milchproduktes, umfassend die Schritte:

1) Zusetzen einer Starterkultur eines Milchsäurebakterienstamms zu einem Milchgrundstoff und
2) Fermentieren der Milch für eine Zeitspanne bis zum Erreichen eines Ziel-pH-Wertes, um ein fermentiertes Milchprodukt zu erhalten,

von

einer Starterkultur von Milchsäurebakterien, umfassend mindestens einen Lactose-defizienten *Streptococcus thermophilus-Stamm,* der in der Lage ist, ein Nicht-Lactose-Kohlenhydrat zu verstoffwechseln, und mindestens einen Lactose-defizienten *Lactococcus* lactis-Stamm, der in der Lage ist, das Nicht-Lactose-Kohlenhydrat zu verstoffwechseln.

12. Verwendung nach Anspruch 11 zum Erhöhen der als Scherbelastung oder Gelfestigkeit gemessenen Textur des fermentierten Milchprodukts im Vergleich zu einer Verwendung einer Starterkultur, die mindestens einen Lactose-positiven *Streptococcus thermophilus-Stamm,* der in der Lage ist, Lactose zu verstoffwechseln, und mindestens einen Lactose-positiven *Lactococcus* lactis-Stamm, der in der Lage ist, Lactose zu verstoffwechseln, umfasst.

**Revendications**

1. Procédé de fabrication d'un produit à base de lait fermenté, comprenant les étapes

    1) d'ajout d'une culture de démarrage de bactéries d'acide lactique comprenant au moins une souche de *Streptococcus thermophilus* déficiente en lactose, qui est capable de métaboliser un glucide autre que le lactose, et au moins une souche de *Lactococcus lactis* déficiente en lactose, qui est capable de métaboliser le glucide autre que le lactose, à une base de lait, et
    2) de fermentation du lait pendant une période de temps jusqu'à ce qu'un pH cible soit atteint pour obtenir un produit à base de lait fermenté,

    dans lequel un glucide non lactique est ajouté à la base de lait au début de l'étape de fermentation.

2. Procédé selon la revendication 1, dans lequel les souches déficientes en lactose sont capables de métaboliser un glucide non lactique choisi dans le groupe constitué du saccharose, du galactose et du glucose.

3. Procédé selon la revendication 1 ou 2, dans lequel le glucide non lactique est ajouté à la base de lait en une quantité mesurée de manière à s'épuiser au pH cible entre 3,2 et 4,8 et donc à entraîner l'arrêt de la croissance des bactéries d'acide lactique et l'arrêt de la fermentation.

4. Procédé selon l'une quelconque des revendications précédentes, dans lequel la souche *Streptococcus thermophilus* déficiente en lactose est choisie dans le groupe constitué :

    (a) de la souche déposée auprès de DSMZ-Deutsche Sammlung von Mikroorganismen und Zellkulturen GmbH, Inhoffenstr. 7B, D-38124 Braunschweig, le 12/06/2014 sous le numéro d'accession DSM 28952 ;
    (b) de la souche déposée auprès de DSMZ-Deutsche Sammlung von Mikroorganismen und Zellkulturen GmbH, Inhoffenstr. 7B, D-38124 Braunschweig, le 12/06/2014 sous le numéro d'accession DSM 28953 ;
    (c) de la souche déposée auprès de DSMZ-Deutsche Sammlung von Mikroorganismen und Zellkulturen GmbH, Inhoffenstr. 7B, D-38124 Braunschweig, le 22/08/2017, sous le numéro d'accession DSM 32599 ; et
    (d) de la souche déposée auprès de DSMZ-Deutsche Sammlung von Mikroorganismen und Zellkulturen GmbH, Inhoffenstr. 7B, D-38124 Braunschweig, le 22/08/2017, sous le numéro d'accession DSM 32600.

5. Procédé selon l'une quelconque des revendications précédentes, dans lequel la souche de *Lactococcus lactis* est choisie dans le groupe constitué d'une souche de *Lactococcus lactis* subsp. *cremoris* et d'une souche de *Lactococcus lactis* subsp. *lactis.*

6. Procédé selon l'une quelconque des revendications précédentes, dans lequel la culture de démarrage contient en outre une ou plusieurs souches choisies dans le groupe constitué de *Lactococcus lactis* subsp. *lactis* biovar. *Diacetylactis,* de *Leuconostoc spp.* et de *Bifidobacterium spp.*

7. Procédé selon l'une quelconque des revendications précédentes, dans lequel le produit à base de lait fermenté produit est choisi dans le groupe constitué du babeurre, du lait aigre, du lait fermenté, de la Smetana, de la crème aigre, de la crème épaisse, de la crème fermentée, de l'ymer, du lactosérum fermenté, du kéfir et du fromage frais, tel que le quark, le tvarog et le fromage à la crème.

8. Procédé selon l'une quelconque des revendications précédentes, dans lequel la souche *Streptococcus thermophilus* déficiente en lactose est choisie dans le groupe constitué

    1) de la souche déposée au DSMZ sous le numéro d'accès DSM 32398,
    2) de la souche déposée au DSMZ sous le numéro d'accès DSM 18882,
    3) de la souche déposée au DSMZ sous le numéro d'accès DSM 32399,
    4) de la souche déposée au DSMZ sous le numéro d'accès DSM 18893,
    5) de la souche déposée au DSMZ sous le numéro d'accès DSM 32601,
    6) de la souche déposée au DSMZ sous le numéro d'accès DSM 32602,
    7) de la souche déposée au DSMZ sous le numéro d'accès DSM 32603,
    8) de la souche déposée au DSMZ sous le numéro d'accès DSM 32604,
    9) de la souche déposée au DSMZ sous le numéro d'accès DSM 32605,
    10) de la souche déposée au DSMZ sous le numéro d'accès DSM 32829,

11) de la souche déposée au DSMZ sous le numéro d'accès DSM 32830, et
12) de la souche déposée au DSMZ sous le numéro d'accès DSM 32832.

9. Composition de bactéries d'acide lactique comprenant au moins une souche de *Streptococcus thermophilus* déficiente en lactose et au moins un *Lactococcus lactis* déficient en lactose.

10. Produit à base de lait fermenté produit par le procédé des revendications 1 à 8.

11. Utilisation dans un procédé de production d'un produit de lait fermenté comprenant les étapes

1) d'ajout d'une culture de démarrage de souche de bactéries d'acide lactique à une base de lait, et
2) de fermentation du lait pendant une période de temps jusqu'à ce qu'un pH cible soit atteint pour obtenir un produit à base de lait fermenté,

d'une culture de démarrage de bactéries d'acide lactique comprenant au moins une souche de *Streptococcus thermophilus* déficiente en lactose, qui est capable de métaboliser un glucide autre que le lactose, et au moins une souche de *Lactococcus lactis* déficiente en lactose, qui est capable de métaboliser le glucide autre que le lactose.

12. Utilisation selon la revendication 11 pour augmenter la texture mesurée en tant que contrainte de cisaillement ou fermeté de gel du produit à base de lait fermenté par rapport à l'utilisation d'une culture de démarrage comprenant au moins une souche de *Streptococcus thermophilus* positive au lactose, qui est capable de métaboliser le lactose, et au moins une souche de *Lactococcus lactis* positive au lactose, qui est capable de métaboliser le lactose.

Fig. 1

**Fig. 2**

Fig. 3

**Acidification at 34 °C**

Legend:
- - - - - DSM32599+DSM32603+0,5%sucrose
——— XPL-30

**Fig. 4**

Fig. 5

Fig. 6

**Fig. 7**

Fig. 8

**Fig. 9**

Fig. 10

**EP 3 675 642 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2957180 A1
- US 2017135360 A1